# EUROPEAN PATENT APPLICATION

(11) **EP 3 763 262 A1**
(43) Date of publication of application: **13.01.2021**
(21) Application number: 20184198.8
(22) Date of filing: 06.07.2020
(51) Int. Cl.: A47K 17/00, A61F 5/453, G09B 19/00, A61F 5/44, A47K 17/02

(54) **BOY URINATION KIT**

(30) Priority: 10.07.2019 US 201916507333
(71) Applicant: Knight, William, Moorpark, California 93021 (US); Knight, Kelly, Moorpark, California 93021 (US)
(72) Inventor: Knight, William, Moorpark, California 93021 (US); Knight, Kelly, Moorpark, California 93021 (US)
(74) Representative: MacLachlan & Donaldson

(57) **Abstract**

A urinary kit and a method that teaches a young boy to stand and direct his urine into a toilet, using a funnel made of material folded around in a circle, rolled into the middle or bottom of the funnel, adhesive used to keep the remaining edge of the funnel, the boy puts his penis into the top of the funnel, and the bottom of the funnel into the toilet. The top of the funnel is foldable to prevent leakage after use, and a label is placed into the toilet for the boy to keep his eyes into the toilet.

## Description

This invention comprises a training urination kit that permits a standing boy to perfectly direct his urine into a toilet while focusing on a visual target.

Using the restroom is a challenge for any boy, potty trained or not, and the individuals who clean after them. The process of a boy urinating, is often referred to as an, "uncontrollable sprinkler" that goes off and leaves everything around it wet, and sometimes smelly and gross. The boys who do this, don't have a reason to act differently. Parents and teachers often resort to yelling and punishing young boys, which is not a positive reinforcement teaching tool.

Because of this situation, many parents are required to clean the restrooms, replace their kids' clothes, and even wash their body, right after a short, unsuccessful use of the toilet, urinal or bowl, often many times per day. This is a time-consuming unpleasant task for parents or pre-school teachers, who have much other things to do. In view of these inherent urination problems, typically associated with boys, at home, school and public restrooms, it would be highly desirable to develop a urination device or kit, easy to use, and readily disposable, which helps parents, teachers, and public establishments, avoid the mess follows boys who use their restrooms.

The present invention is a urination kit, that permits a boy, even as young as 2 years old, to learn how to direct his urine into a toilet while standing. The kit consists of two main components. The first component is a funnel to direct urine in one desirable direction. The second component is a small label that is placed inside the toilet, for a visual urination target.

The funnel is formed from eco friendly material to be readily disposable in an environmentally responsible manner. The label is formed from an eco-friendly or environmentally sound, flushable material.
Fig. 1 is a side view of a boy standing at a toilet;
Fig. 2 is a side view of a boy standing on a stool at a toilet;
Fig. 3 and Fig. 4 are shaped funnels; and
Fig. 5 is a front view of a toilet with a label and a trash bucket with funnels.

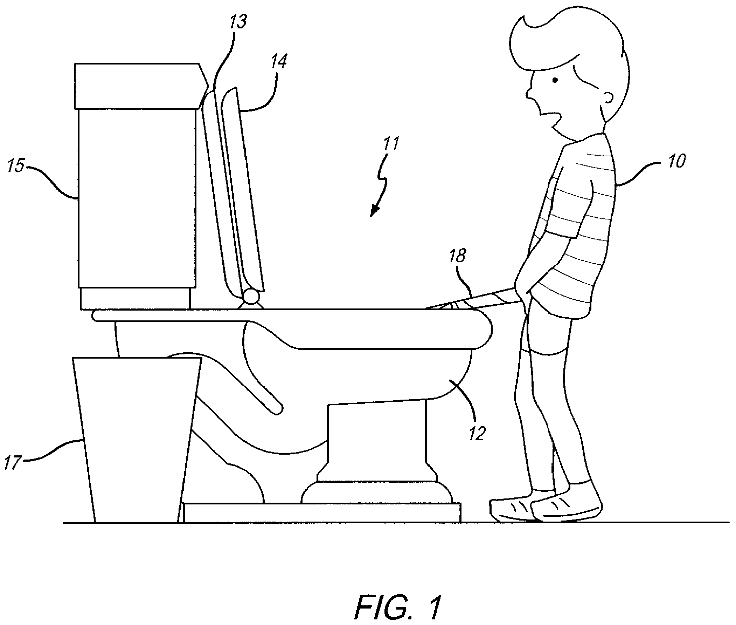

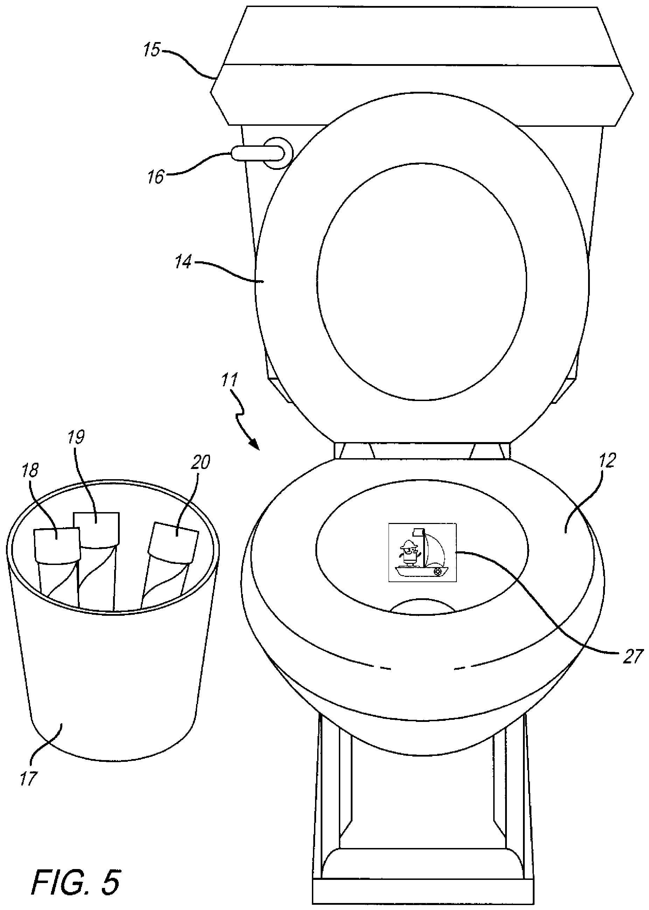

Referring now to the drawings, Fig. 1 in which there is shown a young boy 10, a toilet 11 having a bowl 12, a toilet back 13, and a toilet seat 14. A water tank 15 is set at the back of the toilet 11. A handle 16, shown in Fig. 5, is attached to the water tank 15, to release water into the toilet bowl 12. In Fig. 5, next to the toilet 11 is a trash bucket 17. Inside of trash bucket 17 are three shaped funnels 18, 19 and 20.

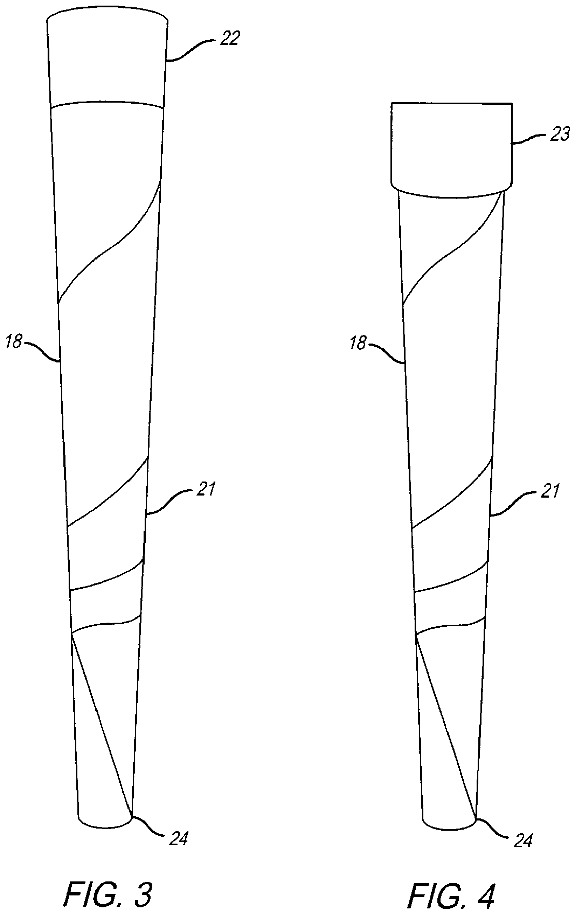

In Fig. 1, a boy 10 is standing in front of a toilet, holding a funnel 18, in which he has put his penis. He holds the funnel 18, to direct his urine into the toilet 11. This is a urination kit that permits a boy to direct his urine into a toilet while standing. In Fig. 3, the funnel 18 is formed from eco-friendly material, discussed below. The funnel 18 material is folded around in a circle 21 so that the remaining edge is rolled into the very bottom 24 of funnel 18, using adhesive at the very bottom 24, to keep the funnel 18, held together, and preventing any adhesive irritation on the boy's penis on the top side 22. The top side 22 of funnel 18 is foldable 23 to prevent leakage, from the top side 22, after the funnel 18 is used. The boy 10 will take the funnel 18, and put his penis into the larger top side 22, the smaller side 24 of funnel 18 going into the toilet bowl 12.

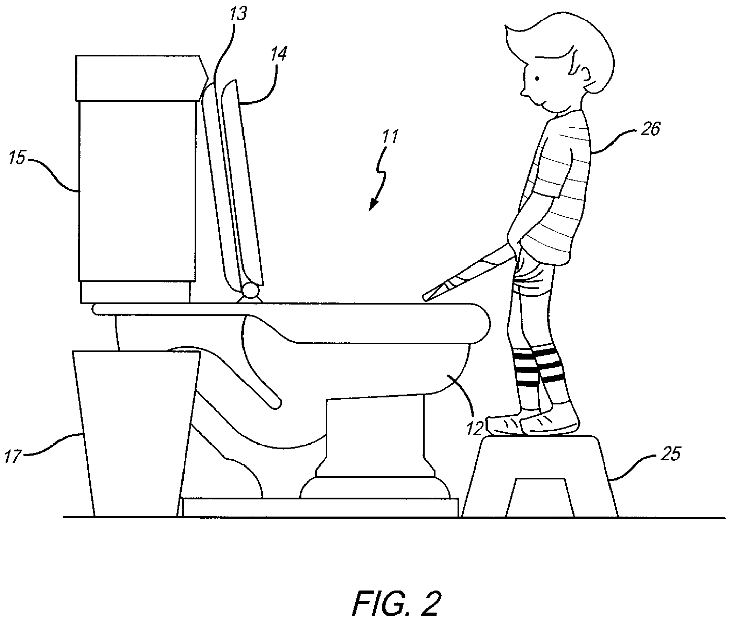

In Fig 2, a smaller boy 26 holding funnel 19 is standing on a stool 25 because the boy 26 is short. He has funnel 19, in which he puts his penis in the larger side 22 of the funnel opening, the small side 24 going into the toilet bowl. Toilet 11 is the same as in Fig. 1, having the bowl 12, the toilet back 13, and the toilet seat 14. A trash bucket 17 is close to toilet 11 but could be placed further from toilet 11. Funnels 18, 19, 20, have been used and thrown into trash bucket 17.

In Fig. 5 there is shown a label 27, which is thrown into the toilet bowl 12. The label 27 can be thrown into the toilet bowl 12, by the parents, or other, and then teach a child to throw it in the toilet bowl 12. Labels 27 can show anything on their face, that a boy will enjoy. In this label 27 it has a pirate ship, or can potentially be any other chosen image. One pirate label 27 is thrown into the toilet by a parent, other, or the boy. Any boy is also given a funnel, and is first teaching the boy how to hold it in place. The boy is also taught to make the label 27 move while urinating, which helps him to keep the boy's eye on the bottom of the toilet bowl 12. Boys as young as two years old can use the funnel. Kids can start using a funnel at age one, with the help of a parent.

The boy learns quickly how to use the funnel 18 and the label 27. The parent, or other, shows the boy how to flush the toilet and will ask the boy to flush while yelling a given word (such as Kamikaze) at the label. The boys aim better when they are aiming at the pirate ship. This procedure has found that the boy is engaged and asks to "pee on pirate ship", when they need to pee. Other of the pirate ship could be animals, cartoon characters, persons and any other items that young boys may enjoy. The parents, or any other third party that provides services to a boy at this age, enjoy an easy, clean, potty training experience. Boys love the game and parents love it even more.

This system handles any stream pressure and therefore prevents splash feedback. If made with paper, it is environmentally sound material, as well as biodegradable and eco-friendly, and maintains its strength well, when urine flows within. It is sanitary and keeps every one's hands clean. It works for potty training for any boy who can stand up without a stool, but can't control the aim or stream pressure of the urinary process. It reduces the frequency of cleaning the toilet and bathroom, whether the urinary process takes place in a residential or commercial location. Food service establishments, such as restaurants, can offer it in their restrooms to prevent children from contaminating their food and prevent the public restroom from getting contaminated.

As required, detailed embodiments of the present invention are disclosed herein; however, it is to be understood that the disclosed embodiments are merely exemplary of the invention that may be embodied in various and alternative forms. The figures are not necessarily to scale; some features may be exaggerated or minimized to show details of particular components. Therefore, specific structural and functional details disclosed herein are not to be interpreted as limiting, but merely as a representative basis for teaching one skilled in the art to variously employ the present invention.

## Claims

1. A urinary kit that teaches a young boy to stand and direct his urine into a toilet, comprising:
a funnel made of material is folded around in a circle so that the remaining edge is rolled into the middle or bottom of the funnel, adhesive is used to keep the remaining edge of the funnel preventing adhesive irritation on the boy's penis, the boy puts his penis into the top of the funnel and the bottom of the funnel is put into the toilet, the top of the funnel is foldable to prevent leakage after use, a label is put into the toilet for the boy to keep his eyes in the toilet.

2. The urinary kit of Claim 1 which the funnel large opening is on the top and the smaller opening is on the bottom.

3. The urinary kit of Claim 1 in which is placed a trash bucket, the boy folds the top of the funnel and is taught to put the funnel into the trash bucket, not into the toilet.

4. The urinary kit of Claim 1 in which there is a stool for a short boy to stand and direct his urine into a toilet.

5. The urinary kit of Claim 1 in which the funnel is made of paper, environmentally sound material, or biodegradable and eco-friendly material.

6. The urinary kit of Claim 1 in which the label can be an item, such as a pirate ship, animals, cartoon characters, persons, and other items that young boys enjoy.

7. The urinary kit of Claim 1 in which the boy is as young as two years.

8. A method for sending a boy's urine into a toilet, the boy standing up, comprising: giving the boy a funnel made of material folded around in a circle, with a larger opening on the top and a smaller opening on the bottom, in which the boy puts his penis in the larger opening and the boy puts the bottom of the funnel into the toilet, the boy's urine going down the funnel into the toilet.

9. The method for sending a boy's urine into a toilet of Claim 8, in which a trash bucket is placed near the toilet, so that the boy sends the funnel into the trash bucket.

10. The method for sending a boy's urine into a toilet of Claim 8, a stool is placed for a short boy to be able to stand and send his urine into the funnel.

11. The method for sending a boy's urine into a toilet of Claim 8, in which a label is put into the toilet, to keep the boy's eyes in the toilet.

12. The method for sending a boy's urine into a toilet of Claim 11, in which the label is a pirate ship, animals, cartoon characters, persons or other items that young boys enjoy.
